# EUROPÄISCHE PATENTSCHRIFT

(11) **EP 0 437 182 B1**
(45) Veröffentlichungstag und Bekanntmachung des Hinweises auf die Patenterteilung: **18.01.1995**
(21) Anmeldenummer: 90811023.2
(22) Anmeldetag: 21.12.1990
(51) Int. Cl.: A61N 5/06

(54) **Faseroptische Vorrichtung für die photodynamische Behandlung von Tumoren**
Fibre optic apparatus for the photodynamic treatment of tumours
Appareil à fibres optiques pour le traitement photodynamique de tumeurs

(30) Priorität: 09.01.1990 CH 59/90
(43) Veröffentlichungstag der Anmeldung: 17.07.1991
(73) Patentinhaber: CIBA-GEIGY AG, 4002 Basel (CH)
(72) Erfinder: Wagnières, Georges, CH-1095 Lutry (CH); Van den Bergh, Hubert, Dr., CH-1376 Goumoens-la-Ville (CH); Monnier, Philippe, Dr., CH-1010 Lausanne (CH)

(56) Entgegenhaltungen:
- DE-A- 2 544 519
- US-A- 4 551 129
- US-A- 4 693 556

## Beschreibung

Die Erfindung betrifft eine faseroptische Vorrichtung für die photodynamische Behandlung von Tumoren, insbesondere in den Atemwegen und der Lunge eines Patienten, mit einer an einen Laser angekoppelten optischen Faser zum Heranführen von Licht an den Ort des Tumors.

Ein derartiges Bronchoskop ist in Chemistry in Britain, Vol. 22, No. 5, May 1986, Hubert van den Bergh, "Light and porphyrins in cancer therapy" beschrieben und gestattet das Aufspüren und Behandeln von bösartigen Lungentumoren, insbesondere an den gabelförmigen Verzweigungen der Bronchien. Bei einer derartigen Behandlung wird einem Patienten Porphyrin injiziert. Nach mehreren Tagen hat das Tumorgewebe wesentlich mehr von diesem Farbstoff aufgenommen als das gesunde Gewebe. Bestrahlt man dann die verdächtige Stelle beispielsweise mit einem Krypton-Laser, der an eine Quarzfaseroptik angeschlossen ist, so erkennt man das Krebsgewebe durch das von ihm ausgehende rote Licht. Neben diesem Effekt, der das Aufspüren von Tumoren ermöglicht, hat Porphyrin noch eine weitere vorteilhafte Eigenschaft, die darin besteht, dass es rotes Licht stark absorbiert, wobei in dem kranken Gewebe eine Reihe photochemischer Reaktionen ausgelöst wird, die das mit dem Porphyrin angereicherte Tumorgewebe abtöten. Das dazu benötigte hochintensive rote Licht kann ebenfalls über eine Quarzfaseroptik zum Tumor geleitet werden, wodurch die Krebszellen bei einer derartigen photodynamischen Therapie selektiv zerstört werden.

In der US-A-4,551,129 ist eine Vorrichtung für die Mikrochirurgie beschrieben, speziell für die Intraokularchirurgie. Diese Vorrichtung umfasst einen hypodermischen Tubus, in welchem ein faseroptischer Lichtleiter angeordnet ist. Am distalen Ende des Lichtleiters ist eine Fokussierlinse vorgesehen. Zwischen dem Lichtleiter und dem Tubus ist ein schmaler Kanal vorgesehen, der von einer Salzlösung durchströmt wird.

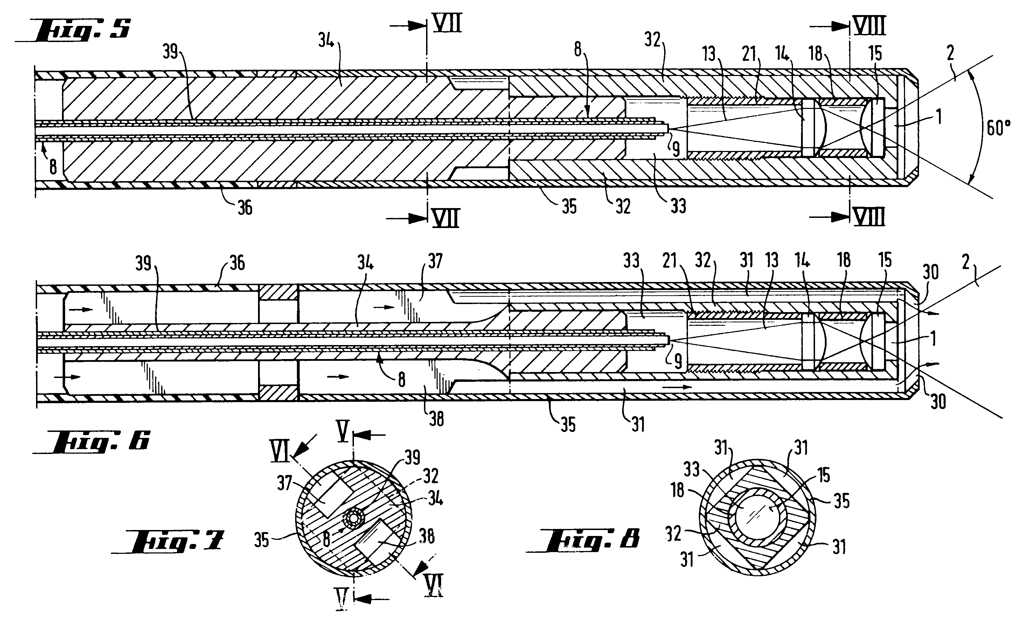

Eine optische Weitwinkelbeleuchtungsvorrichtung, beispielsweise für endoskopische Anwendung, ist aus der DE-OS-25 44 519 bekannt. Allerdings wird bei dieser Vorrichtung nicht die sphärische Aberration von Linsen ausgenützt, um die beleuchtete Fläche homogen und randscharf auszuleuchten. Vielmehr ist der Intensitätsverlauf über der beleuchteten Fläche glockenkurvenförmig, wie der Fig. 5 dieser Offenlegungsschrift zu entnehmen ist. Für eine randscharfe Ausleuchtung der zu bestrahlenden Gewebefläche bei der photodynamischen Therapie ist diese Vorrichtung daher nicht geeignet.

Ausgehend von diesem Stand der Technik liegt der Erfindung die Aufgabe zugrunde, eine faseroptische Vorrichtung der eingangs genannten Art zu schaffen, die es gestattet, zur photodynamischen Behandlung eine Gewebefläche homogen und randscharf auszuleuchten.
Diese Aufgabe wird erfindungsgemäss bei einer faseroptischen Vorrichtung der eingangs genannten Art dadurch gelöst, dass koaxial zu der Licht aussendenden Stirnfläche der Faser ein Linsenpaar mit einer Justierung angeordnet ist, bei der die Randbereichsstrahlen des aus der Stirnfläche der Faser austretenden divergenten Lichtbündels von der ersten Linse des Linsenpaares infolge deren sphärischen Aberration so stark gebrochen werden, dass sie nach radialem überqueren der Mittelstrahlachse des Linsenpaares vor dem Brennpunkt an einer diametral gegenüber der Austrittsstelle aus der Oberfläche der ersten Linse liegenden Stelle in die Oberfläche der zweiten Linse des Linsenpaares eintreten, durch die der Divergenzwinkel der Randbereichsstrahlen verkleinert wird.

Bei einem zweckmässigen Ausführungsbeispiel der Erfindung sind die Linsen des Linsenpaares gleichartige plankonvexe Mikrolinsen, deren Brennweite kleiner als ihr Durchmesser ist und deren aufeinander zuweisende konvexe Seiten einen Abstand voneinander haben, der kleiner als die Brennweite der Mikrolinsen ist.

Der Abstand der Stirnfläche der Faser von der ebenen Oberfläche der ersten Linse beträgt dabei ein Vielfaches der Brennweite der beiden Mikrolinsen. Das Linsenpaar ist in der Nähe des vorderen Endes eines Linsenröhrchens angeordnet, in dessen hinterem Ende die optische Faser befestigt ist.

Gemäss einer Weiterbildung der Erfindung ist vorgesehen, dass das Linsenröhrchen von einem Luftleitröhrchen umgeben ist und dass zwischen dem Linsenröhrchen und dem Luftleitröhrchen Luftkanäle vorgesehen sind, die in Luftdüsen münden, welche an der Stirnseite des Luftleitröhrchens vorgesehen sind.

Weitere zweckmässige Einzelheiten und Ausgestaltungen ergeben sich aus den abhängigen Ansprüchen und der nachfolgenden Beschreibung.

Nachfolgend werden Ausführungsbeispiele der Erfindung anhand der Zeichnung näher beschrieben. Es zeigen:
- Fig. 1: eine faseroptische Vorrichtung mit einem Licht in axialer Richtung aussendenden Mikrodiffusor gemäss der Erfindung,
- Fig. 2: eine schematische Darstellung zur Veranschaulichung des Strahlenganges zwischen der Stirnfläche der optischen Faser und dem Ausgang des Mikrodiffusors,
- Fig. 3: den Verlauf der Funktion des Divergenzwinkels eines Strahles am Ausgang des Miloodiffusors in Abhängigkeit vom Eintrittswinkel in die erste Linse des Linsenpaares am Stirnende des Mikrodiffusors,
- Fig. 4: den Verlauf des Intensitätsprofils im Abstand von 8 mm vor dem Linsenpaar des Mikrodiffusors,
- Fig. 5: ein zweites Ausführungsbeispiel eines Mikrodiffusors gemäss der Erfindung mit Luftdüsen am vorderen Ende im Längsschnitt,
- Fig. 6: den Mikrodiffusor gemäss Fig. 5 im Längsschnitt entlang einer Ebene, die gegenüber der Schnittebene in Fig. 5 um 45 Grad um die Längsachse des Mikrodiffusors verdreht ist,
- Fig. 7: einen Querschnitt durch den Mikrodiffusor entlang der Linie VII-VII in Fig. 5 und
- Fig. 8: einen Querschnitt durch den Mikrodiffusor entlang der Linie VIII-VIII in Fig. 5.

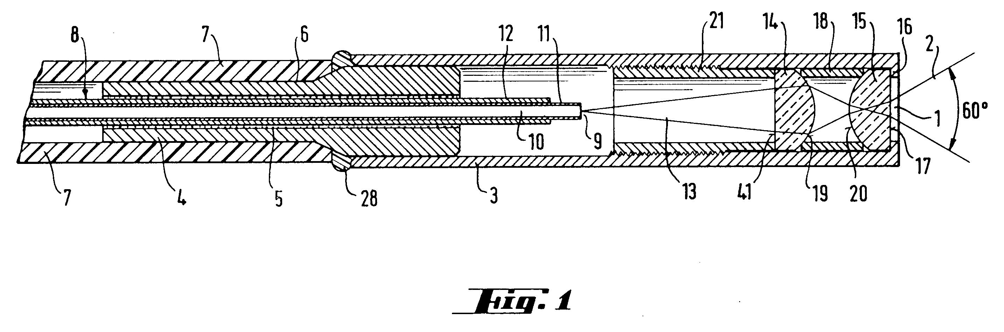

In Fig. 1 erkennt man einen axialen oder frontalen Mikrodiffusor, der es gestattet, im Abstand von 8 mm vor seiner Austrittsblende 1 eine scheibenförmige Fläche mit einem Durchmesser von etwa 10 mm sehr randscharf und homogen zu beleuchten. Das von dem aus der Austrittsblende 1 austretenden Licht beleuchtete Gewebe kann somit infolge der scharfen Kontur des beleuchteten Bereichs sehr selektiv bestrahlt werden, ohne benachbartes Gewebe in unerwünschter Weise ebenfalls zu beleuchten. Ausserdem ist die Lichtenergie in dem Lichtaustrittskegel 2 in hohem Masse homogen verteilt, wobei durch das beleuchtete Gewebe eine weitere Homogenisierung eintritt.

Der in Fig. 1 im Schnitt dargestellte frontale Mikrodiffusor verfügt als Gehäuse über ein Linsenröhrchen oder Edelstahlröhrchen 3 mit einem Aussendurchmesser von 2 mm und einer Länge von 12 mm. In das in Fig. 1 linke Ende des Edelstahlröhrchens 3 ist ein Metallrohr 4 eingesetzt, durch das sich Kanal 5 erstreckt. Der aus dem linken Ende des Edelstahlröhrchens 3 austretende Abschnitt des Metallrohrs 4 verfügt über eine Verjüngung 6, so dass der Aussendurchmesser des Metallrohrs 4 ausserhalb des Edelstahlröhrchens 3 kleiner ist und etwa 1,1 mm beträgt.

Auf das Metallrohr 4 ist ein Kunststoffschlauch, insbesondere ein PTFE-Schlauch 7 aufgezogen, dessen Aussendurchmesser etwa 1,8 mm und dessen Wandstärke etwa 0,35 mm beträgt. Im Bereich der Verjüngung 6 befindet sich zwischen dem PTFE-Schlauch 7 und dem Edelstahlröhrchen 3 eine ringförmige Silikondichtung 28. Der PTFE-Schlauch 7 dient zum Führen des Edelstahlröhrchens 3 beim Vorführen desselben durch die Trachea eines Patienten bis zu den zu untersuchenden und zu behandelnden gabelförmigen Verzweigungen der Bronchien eines Patienten.

Durch das Lumen des PTFE-Schlauches 7 erstreckt sich eine optische Faser 8, die im Kanal 5 des Metallrohres 4 mit Hilfe eines Klebstoffes befestigt ist, so dass die Stirnfläche 9 der Faser 8 im Innern des Edelstahlröhrchens 3 im gewünschten Abstand von der Austrittsblende 1 sicher fixiert ist.

Die optische Faser 8 verfügt über einen Faserkern 10 mit einem Kerndurchmesser von 200 »m, der von einem Mantel 11 mit einem Durchmesser von 280 »m umgeben ist. Eine in Fig. 1 erkennbare Umhüllung 12 dient zum Schutz der optischen Faser 8 und zum Kontakt mit dem Klebstoff im Kanal 5 des Metallrohrs 4.

In das in Fig. 1 nicht dargestellte Einspeiseende der optischen Faser 8 wird bei einer photodynamischen Behandlung eines Lungentumors rotes Laserlicht bei einer Laserleistung von 500 mW und maximal 2 W eingespeist. Das in vielen Moden vorliegende Licht pflanzt sich ausgehend vom Laser über eine geeignete optische Steckeranordnung und die optische Faser 8 durch den PTFE-Schlauch 7 fort und gelangt schliesslich durch das Metallrohr 4 in den Innenraum des Edelstahlröhrchens 3, wo es aus der Stirnfläche 9 als divergentes Lichtbündel 13 mit einem charakteristischen Austrittswinkel von nur 22 Grad mit einer Gaussverteilung austritt.

Das divergente Lichtbündel 13 beaufschlagt zunächst eine erste beispielsweise plankonvexe Mikrolinse 14 und anschliessend eine zweite beispielsweise plankonvexe Mikrolinse 15. Die Mikrolinsen 14, 15 bilden ein Linsenpaar und sind so justiert, dass das wenig randscharfe und inhomogene divergente Lichtbündel 13 an seinen Rändern verschärft und über seine Querschnittsfläche homogenisiert wird. Um eine Transmission des aus den Mikrolinsen 14, 15 bestehenden Linsenpaares von 75 Prozent statt lediglich 60 Prozent zu erhalten, sind die Mikrolinsen 14, 15 mit einer Antireflexschicht aus MgF₂ überzogen.

Wie man in Fig. 1 erkennt, ist am vorderen Ende des Edelstahlröhrchens 3 ein radial nach innen weisender Anschlagflansch 16 vorgesehen, gegen den die zweite plankonvexe Mikrolinse 15 mit ihrer ebenen Seite 17 anliegt. Ein Messingring 18 dient als Abstandhalter zwischen der zweiten Mikrolinse 15 und der ersten Mikrolinse 14. In Fig. 1 erkennt man weiterhin, wie die erste Mikrolinse 14 mit ihrer konvexen Oberseite 19 so ausgerichtet ist, dass diese zur konvexen Oberseite 20 der zweiten Mikrolinse 15 weist. Gegen die ebene Seite 41 der ersten Mikrolinse 14 drückt ein Messingrohr 21 an, das mit einem Gewinde im Innern des Edelstahröhrchens 3 befestigt ist, um das Linsenpaar aus den Mikrolinsen 14 und 15 im Edelstahlröhrchen 3 mit dem durch den Messingring 18 definierten Abstand zu fixieren.

Das Linsenpaar aus den Mikrolinsen 14, 15 verwendet die sphärische Aberration der Linsen, um eine homogene randscharfe ausgeleuchtete Fläche im Lichtaustrittskegel 2 zu gewährleisten.

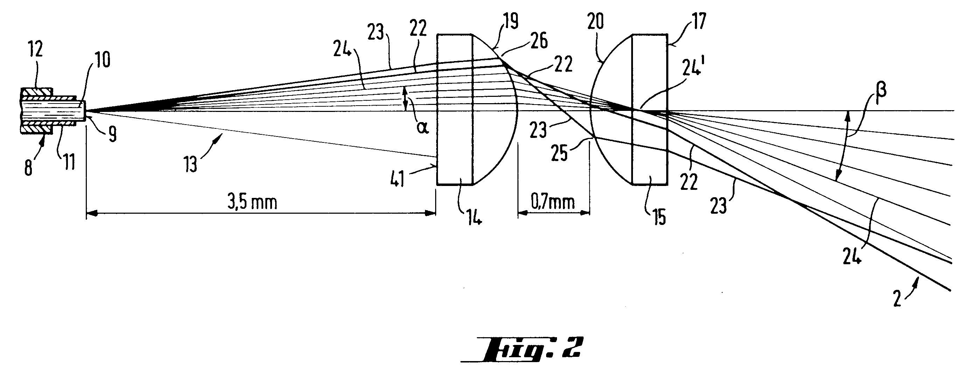

In Fig. 2 ist vergrössert ein Teil des Strahlenganges dargestellt, um die spezielle Justierung und Auswahl der Mikrolinsen 14, 15 zu veranschaulichen.

Links in Fig. 2 erkennt man die optische Faser 8 mit dem Faserkern 10, dem Mantel 11 und der Umhüllung 12. Die numerischen Apertur der Faser beträgt beispielsweise 0,21. Die Dämpfung bei 850 nm ist kleiner als 4 dB/km. Das aus der Stirnseite 9 austretende Licht des divergenten Lichtbündels 13 ist in Fig. 2 durch eine Reihe von Strahlen veranschaulicht wobei der Randstrahl 22 und der äusserste Randstrahl 23 der mit einer grösseren Linienbreite dargestellt ist, für das Verständnis des Strablenganges von besonderer Bedeutung sind. Ein mittlererer Strahl 24 des divergenten Lichtbündels 13 hat den in Fig. 2 eingezeichneten Divergenzwinkel α beim Eintritt in die erste Mikrolinse 14 und den Austrittswinkel β nach dem Durchqueren der zweiten Mikrolinse 15.

Bei den plankonvexen Mikrolinsen 14, 15 handelt es sich um Linsen aus Flintglas (La SF9) mit einem Durchmesser von 1,5 mm und einer Brennweite von 1 mm. Der Brechungsindex des Flintglases beträgt 1,876 bei einer Wellenlänge von 630 nm. Beide Mikrolinsen 14, 15 zeichnen sich aufgrund ihrer grossen Oberflächenkrümmung durch eine sehr starke sphärische Aberration aus, die zur Homogenisierung und Verschärfung des Lichtaustrittskegels 2 ausgenutzt wird. Die sphärische Aberration ist dabei um so grösser, je grösser der Divergenzwinkel α ist. Wie man der Fig. 2 entnehmen kann, werden die Strahlen des Lichtbündels 13 mit einem kleinen Divergenzwinkel durch die Mikrolinse 14 in einen Brennpunkt 24' umgelenkt, der im Körper der zweiten Mikrolinse 15 liegt. Im weiteren Verlauf bilden diese Strahlen mittelachsennahe Strahlen des Lichtaustrittskegels 2.

Der Randstrahl 22 wird jedoch, wie in Fig. 2 zu erkennen ist, aufgrund der sphärischen Aberration so stark gebrochen, dass er die Mittelachse zwischen den Mikrolinsen 14, 15 vor dem Brennpunkt 24' durchquert. Wenn der Divergenzwinkel α so gross wird wie beim äussersten Randstrahl 23, führt die sphärische Aberration zu einer so starken Brechung, dass der gebrochene äusserste Randstrahl 23 die konvexe Oberseite 20 der zweiten Mikrolinse 15 an einer Stelle 25 beaufschlagt, die diametral gegenüber der Stelle 26 liegt, wo der gebrochene äusserste Randstrahl 23 die konvexe Oberseite 19 der ersten Mikrolinse 14 verlässt.

Aufgrund der Orientierung der Oberseite 20 im Bereich der Stelle 25 wird der äusserste Randstrahl 23 in der in Fig. 2 erkennbaren Weise nicht von der Mittelachse zwischen den Mikrolinsen 14, 15 weg gebrochen, sondern in Richtung zur Mittelachse zwischen den Mikrolinsen 14, 15 und damit zur Längsachse des frontalen Mikrodiffusors hin gebrochen. Dies führt dazu, dass der äusserste Randstrahl 23 nach dem Verlassen der ebenen Seite 17 der zweiten Mikrolinse 15 einen Austrittswinkel β hat, der kleiner ist als der Austrittswinkel β von anderen Strahlen im Randbereich mit einem kleineren Divergenzwinkel α, wie zum Beispiel dem Randstrahl 22.

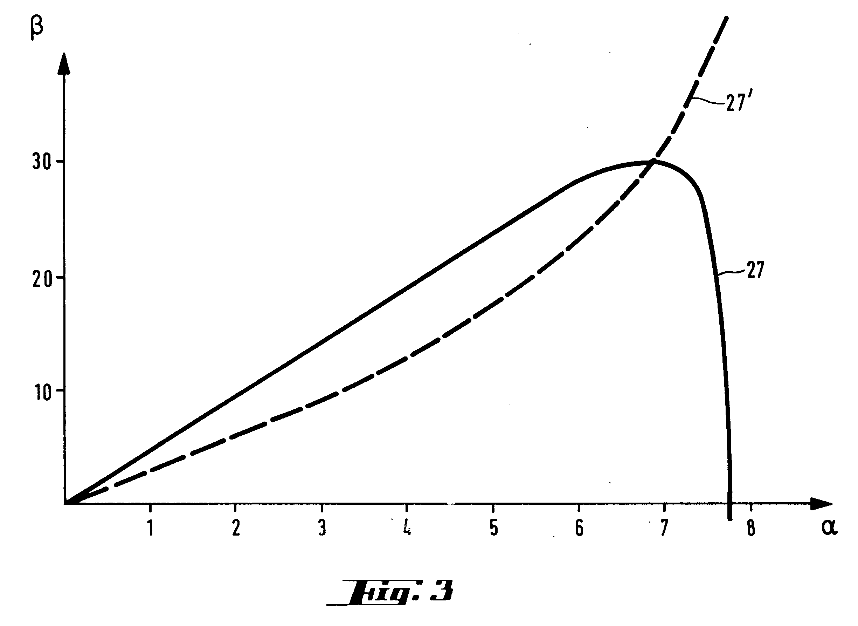

Dieser Zusammenhang ist in Fig. 3 durch den Verlauf einer Kurve 27 dargestellt, die zeigt, wie der Austrittswinkel β mit grösser werdendem Divergenzwinkel α zunächst in etwa linear ansteigt und ab einen Winkel von etwas mehr als 7 Grad steil abfällt. Eine gestrichelte Linie 27' zeigt die Winkelabhängigkeit, die sich ergibt, wenn statt des Linsenpaares aus den Mikrolinsen 14, 15 eine einzige Linse mit einer sphärischen Aberration verwendet wird.

Die Fig. 3, die lediglich den positiven Ast der einen positiven und negativen Divergenzwinkelast aufweisenden Funktion zeigt, veranschaulicht, wie im Lichtaustrittskegel 2 ein maximaler Kegelwinkel von etwa zweimal 30 Grad oder 60 Grad aufgrund der in besonderer Weise ausgenutzten starken sphärischen Aberration der ersten Mikrolinse 14 entsteht. Die Kurve 27 zeigt dabei den Zusammenhang für vom Rand des Kerns der optischen Faser ausgehende Strahlen. Die winkelabhängige Lichtverteilung als Funktion des Divergenzwinkels α im Lichtbündel 13 gegenüber der Längsachse führt dazu, dass Strahlen im Randbereich nur einen maximalen Austrittswinkel β oder Grenzwertwinkel von etwa 30 Grad annehmen, da die Kurve 27 nicht wie die gestrichelt gezeichnete Funktion 28 immer weiter ansteigt, sondern ab dem Grenzwertwinkel wieder abfällt.

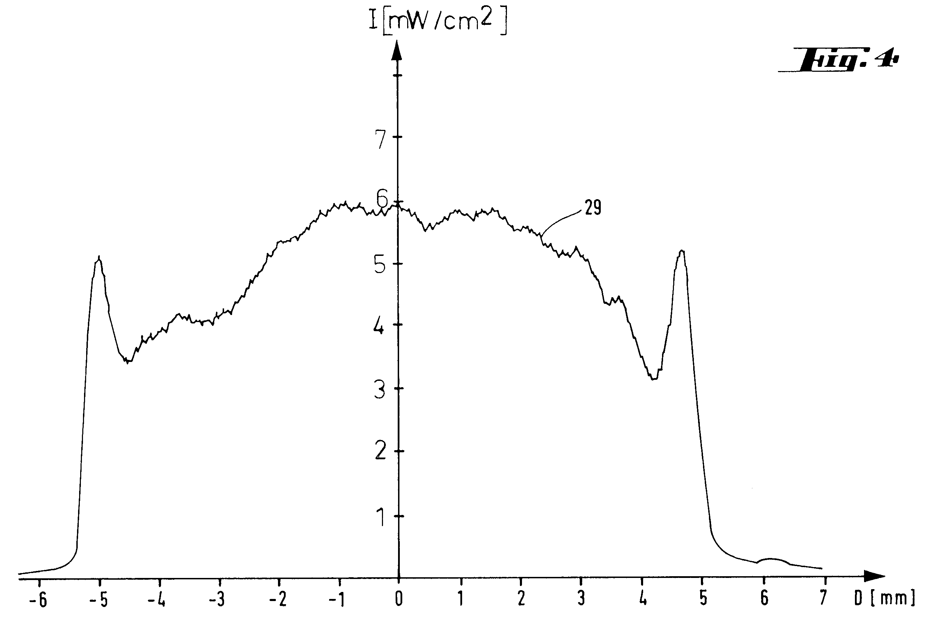

Fig. 4 zeigt ein Intensitätsprofil mit relativen Intensitätseinheiten, wie es sich ergibt, wenn im Abstand von 8 mm vor der zweiten Mikrolinse 15 ein Lichtfleck erzeugt wird. Aus der Abszisse ergibt sich, dass dessen Durchmesser bei dem oben erwähnten Ausführungsbeispiel etwa bei 10 mm liegt, wobei die Intensität am Rand des Lichtflecks oder der Beleuchtungsscheibe abrupt abfällt. Aufgrund der sphärischen Aberration ergeben sich kurz vor dem steilen Abfall Nebenmaxima im Intensitätsprofil 29. Bei einer Laserleistung von 500 mW beträgt die maximale Intensität des Intensitätsprofils 29 etwa 6 mW/cm².

Die Figuren 5 bis 8 veranschaulichen eine Weiterbildung des frontalen oder axialen Mikrodiffusors, wobei die Austrittsblende 1 von Luftdüsen 30 umgeben ist, aus denen Luft zum Wegblasen störender Körperflüssigkeiten austritt. Die Luftdüsen 30 werden über Luftkanäle 31, die in den Fig. 6 und 8 zu erkennen sind, mit Luft gespeist. Die Luftkanäle 31 umgeben ein Vierkantrohr 32 mit einem zylindrischen Innenraum 33 zur Aufnahme der Mikrolinsen 14 und 15, des Messingringes 18 und des Messingrohres 21 in der aus Fig. 1 bereits bekannten Weise. Die optische Faser 8 erstreckt sich durch ein Führungsteil 34, dessen Gestalt sich aus den Fig. 5, 6 und 7 ergibt.

Die bisher beschriebene Anordnung ist von einem Luftleitröhrchen oder Aussenrohr 35 umgeben, das in den Fig. 5, 6, 7 und 8 dargestellt ist. Links erkennt man in den Fig. 5 und 6 einen PTFE-Schlauch 36 mit einem Innendurchmesser von 2,5 mm und einer Wandstärke von 0,25 mm. Durch den PTFE-Schlauch 36 gelangt Spülluft durch einen ersten Luftzufuhrkanal 37 und einen zweiten Luftzufuhrkanal 38, die in den Fig. 6 und 7 zu erkennen sind, in die Luftkanäle 31.

Wie man in Fig. 7 erkennen kann, ist das Führungsteil 34 im wesentlichen als Rundstab ausgebildet, in den zwei axial verlaufende Nuten zur Bildung der Luftzufuhrkanäle 37 und 38 ausgenommen sind. Ein Kanal 39 im Führungsteil 34 hat die gleiche Aufgabe wie der Kanal 5 bei dem in Fig. 1 dargestellten Ausführungsbeispiel des axialen oder frontalen Mikrodiffusors.

Das aus der Stirnfläche 9 austretende Lichtbündel 13 hat bei dem in den Fig. 5 und 6 dargestellten Ausführungsbeispiel die gleichen optischen Eigenschaften und unterliegt den gleichen optischen Verhältnissen wie bei dem Ausführungsbeispiel gemäss Fig. 1. Aus diesem Grunde hat der Lichtaustrittskegel 2 wie bei dem Ausführungsbeispiel gemäss Fig. 1 einen Kegelwinkel von 60 Grad und gestattet das Erzeugen einer homogenen randscharfen ausgeleuchteten Lichtscheibe auf einem Tumorgewebe zur photodynamischen Therapie.

## Patentansprüche

1. Faseroptische Vorrichtung für die photodynamische Behandlung von Tumoren, insbesondere in den Atemwegen und der Lunge eines Patienten, mit einer an einen Laser angekoppelten optischen Faser (8) zum Heranführen von Licht an den Ort des Tumors und mit einem Linsenpaar (14,15), welches koaxial zu der Licht aussendenden Stirnfläche (9) der Faser (8) angeordnet ist und dessen Linsen eine sphärische Aberration aufweisen, dadurch gekennzeichnet, dass das Linsenpaar (14, 15) mit einer Justierung angeordnet ist, bei der die Randbereichsstrahlen (23) des aus der Stirnfläche (9) der Faser (8) austretenden divergenten Lichtbündels (13) von der ersten Linse (14) des Linsenpaares (14,15) infolge deren sphärischer Aberration so stark gebrochen werden, dass sie nach radialem Überqueren der Mittelstrahlachse des Linsenpaares (14, 15) vor dem Brennpunkt (24') an einer diametral gegenüber der Austrittsstelle (26) aus der Oberfläche (19) der ersten Linse (14) liegenden Stelle (25) in die Oberfläche (20) der zweiten Linse (15) des Linsenpaares (14, 15) eintreten, durch die der Divergenzwinkel (β) der Randbereichsstrahlen (23) verkleinert wird.

2. Vorrichtung nach Anspruch 1, dadurch gekennzeichnet, dass die Linsen des Linsenpaares (14,15) gleichartige plankonvexe Mikrolinsen sind, deren Brennweite kleiner als ihr Durchmesser ist und deren aufeinander zuweisende konvexe Seiten (19, 20) einen Abstand voneinander haben, der Kleiner als die Brennweite der Mikrolinsen ist.

3. Vorrichtung nach Anspruch 2, dadurch gekennzeichnet, dass die Stirnfläche (9) der Faser (8) in einem Abstand von der ebenen Oberfläche (41) der ersten Linse (14) angeordnet ist, der ein Vielfaches der Brennweite beträgt.

4. Vorrichtung nach einem der vorstehenden Ansprüche, dadurch gekennzeichnet, dass die Linsen des Linsenpaars (14,15) Mikrolinsen sind und dass das Linsenpaar aus den Mikrolinsen in der Nähe des vorderen Endes eines Linsenröhrchens (3) angeordnet ist, in dessen hinterem Ende die optische Faser (8) befestigt ist.

5. Vorrichtung nach Anspruch 4, dadurch gekennzeichnet, dass das Linsenröhrchen (3, 32) von einem Luftleitröhrchen (35) umgeben ist und dass zwischen dem Linsenröhrchen (3, 32) und dem Luftleitröhrchen (35) Luftkanäle (31, 37, 38) vorgesehen sind, die in Luftdüsen (30) münden, welche an der Stirnseite (1) des Luftleitröhrchens (35) ausgebildet sind.

6. Vorrichtung nach einem der Ansprüche 2 bis 5, dadurch gekennzeichnet, dass die optische Faser (8) einen Kerndurchmesser von 200 »m hat, dass deren numerische Apertur 0,21 ist, dass der Abstand bis zur ersten plankonvexen Mikrolinse (14) 3,5 mm und der Abstand zwischen den Linsen (14, 15) 0,7 mm beträgt, dass die Linsen (14, 15) einen Durchmesser von 1,5 mm haben und dass deren Brennweite 1 mm ist.

7. Vorrichtung nach einem der vorstehenden Ansprüche, dadurch gekennzeichnet, dass die Linsen aus Flintglas mit einem Brechungsindex von 1,876 bei 630 nm hergestellt sind.

## Claims

1. A fibre-optic apparatus for the photodynamic treatment of tumours, especially in the respiratory system and the lungs of a patient, which apparatus has an optical fibre (8) connected to a laser for conveying light to the site of the tumour and a pair of lenses (14, 15) that are arranged coaxially with respect to the light-emitting end face (9) of the fibre (8) and have a spherical aberration, wherein the pair of lenses (14, 15) are so adjusted that the edge rays (23) of the divergent pencil of rays (13) emerging from the end face (9) of the fibre (8) are so greatly refracted by the first lens (14) of the lens pair (14, 15), owing to its spherical aberration, that, after traversing radially the central ray axis of the pair of lenses (14, 15) before the focal point (24'), they enter the surface (20) of the second lens (15) of the lens pair (14, 15) at a site (25) lying diametrically opposite the site of emergence (26) from the surface (19) of the first lens (14), as a result of which the angle of divergence (β) of the edge rays (23) is reduced.

2. An apparatus according to claim 1, wherein the lenses of the lens pair (14, 15) are similar plano-convex microlenses, the focal length of which is smaller than their diameter and the facing convex sides (19, 20) of which are at a distance from one another that is smaller than the focal length of the microlenses.

3. An apparatus according to claim 2, wherein the end face (9) of the fibre (8) is arranged at a distance from the flat surface (41) of the first lens (14) that is a multiple of the focal length.

4. An apparatus according to any one of the preceding claims, wherein the lenses of the lens pair (14, 15) are microlenses and the pair of lenses consisting of the microlenses is arranged near the front end of a lens tube (3) in the rear end of which the optical fibre (8) is secured.

5. An apparatus according to claim 4, wherein the lens tube (3, 32) is surrounded by an air-supply tube (35) and between the lens tube (3, 32) and the air-supply tube (35) there are arranged air ducts (31, 37, 38) that open into air nozzles (30) formed on the front end (1) of the air-supply tube (35).

6. An apparatus according to any one of claims 2 to 5, wherein the optical fibre (8) has a core diameter of 200 »m, its numerical aperture is 0.21, the distance to the first plano-convex microlens (14) is 3.5 mm and the distance between the lenses (14, 15) is 0.7 mm, the lenses (14, 15) have a diameter of 1.5 mm and the focal length thereof is 1 mm.

7. An apparatus according to any one of the preceding claims, wherein the lenses are made of flint glass having a refractive index of 1.876 at 630 nm.

## Revendications

1. Appareil à fibres optiques pour le traitement photodynamique de tumeurs, en particulier dans les voies respiratoires et les poumons d'un patient, l'appareil comportant une fibre optique (8) couplée à un laser pour envoyer de la lumière à l'emplacement de la tumeur et comportant une paire de lentilles (14, 15), qui est disposée coaxialement à la surface frontale (9) de la fibre (8) émettrice de la lumière et dont les lentilles présentent une aberration sphérique, appareil caractérisé en ce que la paire des lentilles (14, 15) est disposée avec un dispositif de mise au point dans lequel les rayons (23) de la zone marginale du faisceau de lumière (13) divergeant sortant de la face frontale (9) de la fibre (8) sont si fortement réfractés par la première lentille (14) de la paire de lentilles (14, 15), en raison de leur aberration sphérique, qu'après avoir traversé radialement l'axe du rayon médian de la paire de lentilles (14, 15) en avant du foyer (24'), ces rayons pénètrent dans la surface (20) de la seconde lentille (15) de la paire de lentilles (14, 15) en un emplacement (25) situé de façon diamétralement opposée à l'emplacement de sortie (26) de la surface (19) de la première lentille (14), grâce à laquelle (15) l'angle de divergence (β) des rayons (23) de la zone marginale est réduit.

2. Appareil selon la revendication 1, caractérisé en ce que les lentilles de la paire (14, 15) de lentilles sont des microlentilles planes-convexes d'un même genre, dont la distance focale est inférieure au diamètre et dont les faces convexes (19, 20) qui se font face mutuellement sont séparées par une distance inférieure à la distance focale des microlentilles.

3. Appareil selon la revendication 2, caractérisé en ce que la surface frontale (9) de la fibre (8) est disposée à une distance de la surface plane (41) de la première lentille (14) qui constitue un multiple de la distance focale.

4. Appareil selon l'une quelconque des revendications précédentes, caractérisé en ce que les lentilles de la paire (14, 15) de lentilles sont des microlentilles, et en ce que la paire de lentilles, formée par les microlentilles, est disposée au voisinage de l'extrémité avant d'un petit tube (3) pour lentilles, à l'extrémité arrière duquel la fibre optique (8) est fixée.

5. Appareil selon la revendication 4, caractérisé en ce que les petits tubes (3, 32) pour lentilles sont entourés d'un petit tube (35) à air et en ce qu'entre les petits tubes (3, 32) pour lentilles et le petit tube (35) conducteur d'air sont prévus des canaux (31, 37, 38) à air, qui débouchent dans des buses (30) à air, lesquelles sont formées sur le côté frontal (1) du petit tube (35) d'acheminement d'air.

6. Appareil selon l'une des revendications 2 à 5, caractérisé en ce que la fibre optique (8) a un diamètre de coeur de 200 »m ; en ce que son ouverture numérique est de 0,21 ; en ce que la distance jusqu'à la première microlentille (14) plane-convexe est de 3,5 mm et la distance entre les lentilles (14, 15) vaut 0,7 mm ; en ce que les lentilles (14, 15) ont un diamètre de 1,5 mm et en ce que la distance focale est de 1 mm.

7. Appareil selon l'une des revendications précédentes, caractérisé en ce que les lentilles sont réalisées en du verre "flint" ayant un indice de réfraction de 1,876 à 630 nm.
